# EUROPEAN PATENT APPLICATION

(11) **EP 4 353 234 A1**
(43) Date of publication of application: **17.04.2024**
(21) Application number: 22804087.9
(22) Date of filing: 20.05.2022
(51) Int. Cl.: A61K 31/519, A61P 25/00

(54) **USE OF PYRROLOPYRIMIDINE COMPOUND**

(30) Priority: 21.05.2021 CN 202110557248; 09.05.2022 CN 202210503501
(71) Applicant: GUANGZHOU JOYO PHARMATECH CO., LTD, Guangzhou, Guangdong, 510663 (CN)
(72) Inventor: LI, Yongguo, Guangzhou, Guangdong 510663 (CN); CHEN, Xiaoning, Guangzhou, Guangdong 510663 (CN)
(74) Representative: Greaves Brewster LLP
(86) International application number: PCT/CN2022/094256
(87) International publication number: WO 2022/242768

(57) **Abstract**

Provided is the use of a pyrrolopyrimidine compound. The structure of the pyrrolopyrimidine compound is as represented by formula I, which compound has the effect of inhibiting the phosphorylation of STAT, and has a good prevention or treatment effect on suppurative myelitis, acute myelitis, encephalomyelitis or autoimmune diseases.

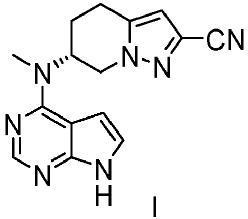

## Description

The present application claims the right of the priorities of Chinese patent application No. 2021105572488, filed on May 21, 2021, and Chinese patent application No. 2022105035016, filed on May 9, 2022. The contents of the above Chinese patent applications are incorporated herein by reference in its entirety.

### TECHNICAL FIELD

The present disclosure relates to a use of a pyrrolopyrimidine compound.

### BACKGROUND

Central nervous system demyelinating disease is an autoimmune disorder mainly characterized by multifocal and inflammatory demyelination in the central nervous system (CNS). Clinically, it is primarily marked by recurrent attacks, multiple remissions, and relapses. Multiple sclerosis is a relatively common form of central nervous system demyelinating disease encountered in clinical practice.

Multiple sclerosis (MS) is the most common type of central nervous system demyelinating disease and is also the most prevalent non-traumatic neurological disorder among young adults. There are currently over two million patients worldwide. As awareness of the disease has grown and diagnostic technologies have improved, the rate of MS diagnosis in China has been gradually increasing year by year. However, due to the complexity of neurological disorders and the unknown etiology of multiple sclerosis, there are still no effective prevention or treatment methods for MS. At present, the medications commonly used for treating multiple sclerosis are primarily steroids. However, steroids have significant side effects, including disturbances in the metabolism of water, salt, glucose, protein, and fat. They may also lead to obesity, excessive hair growth, weakness, etc. In some cases, the symptoms of MS include numbness or weakness in one or multiple parts of the body, partial or complete loss of vision, persistent double vision, tingling or pain, Lhermitte's sign, tremors, slurred speech, fatigue, dizziness, and impairment of bowel and bladder function.

Experimental autoimmune encephalomyelitis (EAE) has been around for roughly 50 years since its initial establishment (Kabat et al., 1947). EAE can be an effective model for studying the role of the immune system in inflammatory demyelinating lesions within the CNS by activating the immune response generated by autoreactive T cells against CNS myelin. Its typical pathological features include: (1) perivascular infiltration of inflammatory cells (primarily lymphocytes, macrophages, and activated microglia); (2) demyelination in the lesion area, characterized by myelin destruction along with axonal preservation (Rao and Segal, 2004). The immunoinflammatory responses, clinical manifestations, and histopathological changes in EAE bear a striking resemblance to human MS disease (Sosa and Forsthuber, 2011). Therefore, it is currently the most widely used animal model in MS research (Kipp et al., 2012). Both MS and EAE feature demyelinating lesions in the CNS white matter with infiltration of T cells, macrophages, and B cells. Within these lesions, foam-like macrophages formed by phagocytosis of hydrophobic myelin are considered to be active lesions. Oligoclonal IgG can be found in the cerebrospinal fluid (CSF) of both EAE and MS patients. The difference between the two lies in the fact that the pathogenesis of the latter also involves genetic, epigenetic, and environmental factors (including the interactions between infections and the immune system) (Becher et al., 2016).

Therefore, the research and development of safe and effective medications for the prevention and treatment of multiple sclerosis, aimed at eliminating neuro-immune inflammation abnormalities within patients, hold significant implications for the clinical treatment of multiple sclerosis.

### CONTENT OF THE PRESENT INVENTION

The technical problem that the present disclosure aims to solve is to find a compound that has good preventative and/or therapeutic effects on purulent myelitis, acute myelitis, encephalomyelitis, or autoimmune diseases. To this end, the present disclosure provides a use of a pyrrolopyrimidine compound.

The present disclosure provides a use of a compound of formula I or a pharmaceutically acceptable salt thereof in the manufacture of a STAT phosphorylation inhibitor:

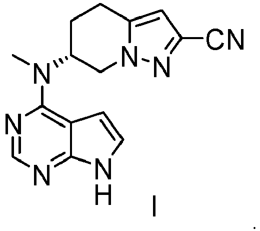

The present disclosure also provides a use of a compound of formula I or a pharmaceutically acceptable salt thereof in the manufacture of a medicament for treating and/or preventing purulent myelitis, acute myelitis, or encephalomyelitis characterized by abnormally elevated levels of STAT phosphorylation:

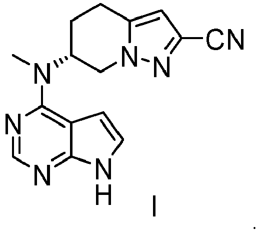

The present disclosure also provides a use of a compound of formula I or a pharmaceutically acceptable salt thereof in the manufacture of a medicament for treating and/or preventing autoimmune diseases characterized by abnormally elevated levels of STAT phosphorylation:

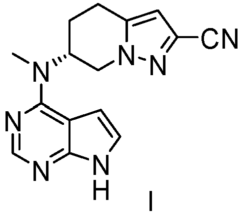

The present disclosure also provides a use of a compound of formula I or a pharmaceutically acceptable salt thereof in the manufacture of a medicament for treating and/or preventing purulent myelitis, acute myelitis, or encephalomyelitis:

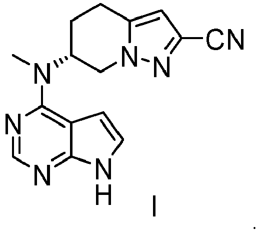

The present disclosure also provides a use of a compound of formula I or a pharmaceutically acceptable salt thereof in the manufacture of a medicament for treating and/or preventing autoimmune diseases:

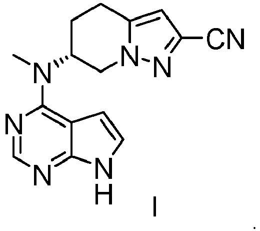

The present disclosure also provides a method for treating and/or preventing purulent myelitis, acute myelitis, or encephalomyelitis in a subject, comprising administering to the subject a therapeutically effective amount of a compound of formula I or a pharmaceutically acceptable salt thereof;

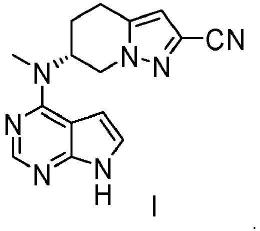

The present disclosure also provides a method for treating and/or preventing autoimmune diseases in a subject, comprising administering to the subject a therapeutically effective amount of a compound of formula I or a pharmaceutically acceptable salt thereof;

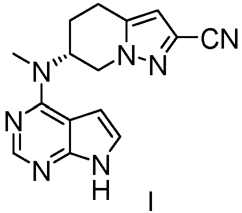

The present disclosure also provides a method for inhibiting STAT phosphorylation in a subject, comprising administering to the subject a therapeutically effective amount of a compound of formula I or a pharmaceutically acceptable salt thereof:

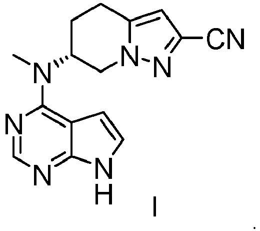

The present disclosure also provides a method for treating and/or preventing purulent myelitis, acute myelitis, or encephalomyelitis characterized by abnormally elevated levels of STAT phosphorylation in a subject, comprising administering to the subject a therapeutically effective amount of a compound of formula I or a pharmaceutically acceptable salt thereof;

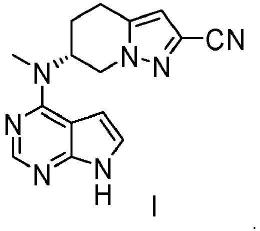

The present disclosure also provides a method for treating and/or preventing autoimmune diseases characterized by abnormally elevated levels of STAT phosphorylation in a subject, comprising administering to the subject a therapeutically effective amount of a compound of formula I or a pharmaceutically acceptable salt thereof;

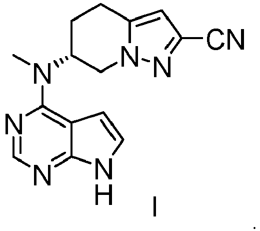

In some embodiments, the STAT phosphorylation may be one or more than one of STAT1 phosphorylation, STATS phosphorylation, and STATS phosphorylation, such as STATS phosphorylation.

In some embodiments, the autoimmune disease may be an autoimmune disease of the central nervous system or an autoimmune disease of the optic nervous system; the autoimmune disease of the central nervous system may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

In some embodiments, the encephalomyelitis may be acute disseminated encephalomyelitis, subacute necrotizing myelitis, acute necrotizing hemorrhagic encephalomyelitis, pediatric acute disseminated encephalomyelitis, or tuberculous myelitis.

In some embodiments, the autoimmune disease characterized by abnormally elevated levels of STAT phosphorylation may be an autoimmune disease of the central nervous system or an autoimmune disease of the optic nervous system; the autoimmune disease of the central nervous system characterized by abnormally elevated levels of STAT phosphorylation may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

In some embodiments, among the encephalomyelitis, purulent myelitis, or acute myelitis characterized by abnormally elevated levels of STAT phosphorylation, the encephalomyelitis may be acute disseminated encephalomyelitis, subacute necrotizing myelitis, acute necrotizing hemorrhagic encephalomyelitis, pediatric acute disseminated encephalomyelitis, or tuberculous myelitis.

In some embodiments, the compound of formula I or the pharmaceutically acceptable salt thereof is one of the active ingredients or the sole active ingredient of the inhibitor or the medicament.

In some embodiments, a therapeutically effective amount of the compound of formula I or the pharmaceutically acceptable salt thereof can be administered to a subject in need. The medicament can be administered by any suitable method, such as oral administration.

In some embodiments, the compound of formula I or the pharmaceutically acceptable salt thereof may further include a pharmaceutically acceptable excipient, such as a tablet and a subcutaneous injection.

In some embodiments, the compound of formula I or the pharmaceutically acceptable salt thereof can be administered at a dose of 1 to 120 mg/kg/d, preferably 3 to 100 mg/kg/d, for example, 3 mg/kg/d, 6 mg/kg/d, 10 mg/kg/d, 20 mg/kg/d, 40 mg/kg/d, 60 mg/kg/d, or 90 mg/kg/d.

In some embodiments, the compound of formula I or the pharmaceutically acceptable salt thereof can be administered at a single dose of 1 to 400 mg, for example, 3 mg, 6 mg, 9 mg, 100 mg, or 400 mg.

In some embodiments, the compound of formula I or the pharmaceutically acceptable salt thereof can be administered at a frequency of once a day, twice a day, or three times a day, preferably once a day or twice a day.

The present disclosure also provides a pharmaceutical composition for treating and/or preventing purulent myelitis, acute myelitis, encephalomyelitis, or autoimmune diseases in a subject, comprising a compound of formula I or a pharmaceutically acceptable salt thereof, as well as a pharmaceutical excipient.

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "STAT phosphorylation inhibitor" can be used in mammalian organisms *in vivo*; it can also be used *in vitro,* primarily for experimental purposes. For example, it can serve as a standard or control sample for comparison, or be incorporated into a kit according to conventional methods in this field to provide rapid detection of STAT phosphorylation inhibitory effects.

The term "autoimmune disease characterized by abnormally elevated levels of STAT phosphorylation" means that the autoimmune disease exhibits higher than normal levels of STAT phosphorylation. In the present disclosure, the compound of formula I can have preventative and/or therapeutic effects on autoimmune diseases by inhibiting STAT phosphorylation.

The term "purulent myelitis, acute myelitis, or encephalomyelitis characterized by abnormally elevated levels of STAT phosphorylation" means that the purulent myelitis, acute myelitis, or encephalomyelitis exhibits higher than normal levels of STAT phosphorylation. In the present disclosure, the compound of formula I can have preventative and/or therapeutic effects on purulent myelitis, acute myelitis, or encephalomyelitis by inhibiting STAT phosphorylation.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt prepared by the compound of the present disclosure with a relatively nontoxic, pharmaceutically acceptable acid or base. When the compound of the present disclosure contains a relatively acidic functional group, a base addition salt can be obtained by bringing the neutral form of the compound into contact with a sufficient amount of a pharmaceutically acceptable base in a pure solution or a suitable inert solvent. Pharmaceutically acceptable base addition salts include, but are not limited to, lithium salt, sodium salt, potassium salt, calcium salt, aluminum salt, magnesium salt, zinc salt, bismuth salt, ammonium salt, and diethanolamine salt. When the compound of the present disclosure contains a relatively basic functional group, an acid addition salt can be obtained by contacting the neutral form of the compound with a sufficient amount of a pharmaceutically acceptable acid in a pure solution or a suitable inert solvent. The pharmaceutically acceptable acid includes inorganic acid, and the inorganic acid includes, but is not limited to, hydrochloric acid, hydrobromic acid, hydroiodic acid, nitric acid, carbonic acid, phosphoric acid, phosphorus acid, sulfuric acid, etc. The pharmaceutically acceptable acid includes organic acid, and the organic acid includes, but is not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, aconitic acid, oleic acid, tannic acid, pantothenic acid, tartaric acid, ascorbic acid, gentisic acid, fumaric acid, gluconic acid, saccharic acid, formic acid, ethanesulfonic acid, pamoic acid (i.e. 4,4'-methylene-bis(3-hydroxy-2-naphthoic acid)), amino acids (such as glutamic acid and arginine), etc. When the compound in the present disclosure contains relatively acidic and relatively basic functional groups, it can be converted into a base addition salt or an acid addition salt. For more details, please refer to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "treat/treatment" refers to therapeutic interventions. In the context of specific diseases, "treat/treatment" refers to: (1) alleviating one or more biological manifestations of a disease or condition; (2) interfering with (a) one or more points in the biological cascade leading to or causing the condition or (b) one or more biological manifestations of the condition; (3) improving one or more symptoms, effects, or side effects associated with the condition, or one or more symptoms, effects, or side effects associated with the condition or its treatment, or (4) slowing the progression of one or more biological manifestations of the disease or condition.

The term "prevent/prevention" refers to a reduction in the risk of acquiring or developing a disease or disorder.

The term "subject" refers to any animal that is to be or has been administered the compound in accordance with embodiments of the present disclosure, with mammals being preferred, and humans being most preferred. The term "mammal" includes any mammal. Examples of mammals include, but are not limited to, cattle, horses, sheep, pigs, cats, dogs, mice, rats, rabbits, guinea pigs, monkeys, humans, etc., with humans being most preferred.

Without departing from the common understanding in this field, the aforementioned preferred conditions can be freely combined to obtain the preferred examples of the present disclosure.

The reagents and raw materials used in the present disclosure are all commercially available.

The positive progressive effect of the present disclosure lies in the discovery that the compound of formula I has the effect of inhibiting STAT phosphorylation and has a favorable preventative and/or therapeutic effect on purulent myelitis, acute myelitis, encephalomyelitis, or autoimmune diseases.

### BRIEF DESCRIPTION OF THE DRAWINGS

Figure 1 shows the effect of repeated administration of test compound I on the clinical assessment of EAE in mice. (Data are presented as mean or mean ± SEM (standard error of the mean), *p<0.05, **p<0.01, ***p<0.001 VS G2).
Figure 2 shows the effect of repeated administration of test compound I on the spinal cord pathology scores in mice. (Data are presented as mean ± SEM, *p<0.05, **p<0.01, ***p<0.001, ****p<0.0001 VS G2).
Figure 3 shows the effect of repeated administration of test compound I on the STATS phosphorylation levels of spinal cord. (Data are presented as mean ± SEM, **p<0.01, ***p<0.001 VS G2).
Figure 4 shows the effect of 24-hour co-incubation of compound I on cell viability in NMO-IgG induced astrocyte injury model.
Figure 5 shows the effect of compound I on IL-6 release caused by NMO-IgG in astrocytes.

### DETAILED DESCRIPTION OF THE PREFERRED EMBODIMENT

The present disclosure will be further illustrated by way of examples below, but the present disclosure should not be limited to the scope of these examples. The experimental methods for which the specific conditions are not specified in the following examples are selected according to the conventional methods and conditions, or according to the commodity instructions. The solvents and reagents used in the present disclosure are commercially available.

Compound I was provided by Guangzhou JOYO Pharmatech Co., Ltd. and was prepared according to the preparation method described in patent PCT/CN2020094534.

### Example 1: Study on inhibitory effects of compound I on CCL5 and NO secretion by BV-2 cell line

### 1. Experimental materials and methods

### (1) Cell line

**Table 1: Cell line**

| Cell name | Tissue type | Culture characteristics | Culture medium | Number of cells per well |
|---|---|---|---|---|
| BV-2 | Mouse glioma | Adherent | RPMI 1640 + 10% FBS | 12000 |

| | | | | |
|---|---|---|---|---|
| Note: 1. Cell culture conditions were set at 37°C, 5% CO₂, and 95% humidity. 2. IC₅₀ value: The concentration of inhibitor at which 50% inhibition is achieved. 3. BV-2 was purchased from Nanjing Cobioer Biosciences Co. Ltd. 4. The percentage in "10% FBS" in Table 1 is expressed as a volume percentage. | | | | |

### (2) Reagents

1) RPMI1640 culture medium (purchased from Gibco, product number: C22400500BT); 2) FBS (fetal bovine serum) (purchased from ExCell, product number: FND500); 3) DMSO (purchased from Sigma, product number: D2650); 4) LPS lipopolysaccharide (purchased from Sigma, product number: L4391); 5) Mouse IFN-gamma Protein (purchased from R&D, product number: 485-MI-100); 6) CellTiter-Glo (CTG) (purchased from Promega, product number: G7572); 7) Mouse CCL5/RANTES DuoSet ELISA (R&D, product number: DY478-05); 8) Griess Reagent System (Promega, product number: G2930).

### (3) Test sample and positive control

**Table 2: Information on test sample and positive control**

| **Compound** | **Batch number** | **Purity (%)** | **Molecular weight** | **Storage conditions** | **Supplier** |
|---|---|---|---|---|---|
| Compound I | H22005-035-P1 | 98.1 | 293 | 2 to 8°C - sealed/ protected from light | provided by Guangzhou JOYO Pharmatech Co., Ltd. |
| Siponimod | T6403 | 99.1 | 516.6 | -20°C | Topscience Co., Ltd. |

Compound I and Siponimod were weighed and dissolved in DMSO (dimethyl sulfoxide) to make a 10 mM stock solution, which was stored at -20°C for further use.

### (4) CTG method for measuring compound cell proliferation, and detection of CCL5 and NO in supernatant

Specific steps are as follows:
Step 1: BV-2 cells in the exponential growth phase were collected, and viable cells were counted using a Vi-Cell XR cell counter. The cell suspension was adjusted to an appropriate concentration with the culture medium. 160 µL of cell suspension was added to each well of a 96-well cell culture plate, resulting in a final cell concentration of 12,000 cells per well. The culture plate was incubated for 24 hours in a 37°C, 5% CO₂ incubator.
Step 2: A 10 mM DMSO compound stock solution was diluted at a ratio of 1:4 using DMSO to prepare a series of 1000X test compound solutions. These 1000X test compound solutions were further diluted at a ratio of 1:100 using cell culture medium to prepare a series of 10X test compound solutions. Finally, the 96-well plate that had been plated with cells the day before was taken out. According to the experimental well plate loading design, 20 µL of the corresponding 10X solution was added to each well, with three duplicate wells for each drug concentration. The final concentrations of the test compounds used in the experiment (i.e., 1X solutions) have a range of 10, 2.5, 0.625, 0.156, 0.0391, 0.0098, and 0.0024 µM. The final DMSO concentration in each well was 0.1%.

After the compound was added, the 96-well plate was incubated for half an hour in the 37°C, 5% CO₂ incubator.

After the pre-incubation was completed, 20 µL of culture medium containing 100 ng/mL LPS and 100 ng/mL IFN-γ (final concentrations of 10 ng/mL LPS and 10 ng/mL IFN-γ) was added to each well to induce the activation of BV-2 cells. The culture plate was then incubated for 24 hours in the 37°C, 5% CO₂ incubator.

Step 3: After 24 hours of treatment with the compound, the culture plate was centrifuged at 1200 rpm for 5 minutes. The supernatant was pipetted into another plate, which was sealed with tinfoil and stored at -20°C for future ELISA and Griess reagent system assays. The cell culture plate from which the supernatant had been removed was immediately used for cell proliferation assays using the CellTiter-Glo (CTG) method.

Step 4: In the cell culture plate from which the supernatant had been removed, 100 µL of PBS was added to each well. According to the instructions of the CellTiter-Glo (CTG) kit, 50 µL of pre-melted and room-temperature balanced CTG solution was added to each well. The plate was then mixed for 2 minutes using a microplate shaker. After letting it stand at room temperature for 10 minutes, the fluorescence signal value was measured using an Envision 2104 plate reader.

Step 5 (NO and CCL5 detection): The CCL5 levels in the supernatant were detected using an ELISA kit (Mouse CCL5/RANTES DuoSet ELISA; R&D; DY478-05), following the instructions provided with the kit. The nitric oxide (NO) levels in the supernatant were detected using the Griess Reagent System (Promega; G2930), following the instructions provided with the kit.

### (5) Data analysis

### 1) CTG data analysis

Step 1: Using the CTG fluorescence signal values, the percentage of surviving cells (surviving cells %) at each test concentration relative to the blank (DMSO) group was calculated. The formula was: surviving cells% = fluorescence value of the test concentration group/fluorescence value of the blank group.

Step 2: the percentage of surviving cells was plotted against the logarithm of the compound concentration. Nonlinear regression was used to fit the curve, and the formula "log(inhibitor) vs. response -- Variable slope" in the software GraphPad Prism 5 was employed to calculate the IC₅₀ value.

Step 3: The formula for calculating the inhibition rate was: inh. (%) = f fluorescence value of the blank group - fluorescence value of the test concentration group}/fluorescence value of the blank (DMSO) group.

### 2) CCL5 and NO data analysis

Step 1: Firstly, the optical density values obtained from ELISA and Griess tests were used to plot a standard curve and calculate the absolute concentrations of each sample. The absolute concentration was then calibrated using the CTG fluorescence value corresponding to each sample to obtain the calibrated concentration. The formula for calculating the calibrated concentration was: calibrated concentration = absolute concentration/CTG fluorescence value of the same sample.

Step 2: Based on the calibrated concentration of each sample, the relative percentage of CCL5 or NO secretion (i.e., CCL5 production (%) or NO production (%)) at each test concentration relative to the blank (DMSO) group was calculated. The formula for calculating the relative percentage was: relative percentage% = (calibrated concentration of the test concentration group)/(calibrated concentration of the blank group).

Step 3: Finally, the relative percentage of CCL5 secretion or the relative percentage of NO secretion was plotted against the logarithm of the compound concentration. Nonlinear regression was used to fit the curve, and the formula "log(inhibitor) vs. response -- Variable slope" in the software GraphPad Prism 5 was employed to calculate the IC₅₀ value.

Step 4: The formula for calculating the inhibition rate was: inh. (%) = {(calibrated concentration of the blank group) - (calibrated concentration of the test concentration group)}/(calibrated concentration of the blank group).

### (6) Experimental results

**Table 3: IC₅₀ and maximum inhibition rate of compound I and Siponimod on BV-2 cell proliferation**

| Cell line | Compound I | | Siponimod | |
|---|---|---|---|---|
| | IC₅₀ (µM) | Maximum inhibition rate (%) | IC₅₀ (µM) | Maximum inhibition rate (%) |
| BV-2 | NA | -46.39% | NA | 4.44% |

Conclusion: After a 24-hour co-incubation with BV-2 cells, compound I showed no inhibitory effect on cell proliferation (Table 3), with the highest test concentration being 10 µM. Additionally, when the concentration of compound I was greater than 0.1 µM, there was an increase in cell numbers compared to the blank (DMSO) well, with the maximum rate of increase reaching 46.39% (Table 3), which was close to the cell number under conditions without LPS/IFNγ, suggesting that compound I may enhance the viability of activated BV-2 cells. Siponimod also showed no significant inhibitory effect on cell proliferation at any test concentration, with the maximum inhibition rate being 4.44%.

**Table 4: IC₅₀ and maximum inhibition rate of compound I and Siponimod on mouse CCL5 secretion by BV-2 cell line**

| | | | | |
|---|---|---|---|---|
| Effect of compounds on CCL5 secretion by BV-2 | Compound I | | Siponimod | |
| | IC₅₀ (µM) | Maximum inhibition rate (%) | IC₅₀ (µM) | Maximum inhibition rate (%) |
| | 0.863 | 80.29 | >10 | 35.16 |

Conclusion: The CCL5 ELISA results showed that after co-incubation with BV-2 cells for 24 hours, compound I had an IC₅₀ of 0.863 µM and a maximum inhibition rate of 80.29% (Table 4). Under the same conditions, Siponimod had an IC₅₀ greater than 10 µM and a maximum inhibition rate of 35.16% (Table 4).

**Table 5: IC₅₀ and maximum inhibition rate of compound I and Siponimod on NO secretion by BV-2 cell line**

| | | | | |
|---|---|---|---|---|
| Effect of compounds on NO secretion by BV-2 | Compound I | | Siponimod | |
| | IC₅₀ (µM) | Maximum inhibition rate (%) | IC₅₀ (µM) | Maximum inhibition rate (%) |
| | 0.487 | 100.00 | >10 | 7.71 |

Conclusion: The results from the Griess reagent system showed that after a 24-hour co-incubation with BV-2 cells, compound I had an IC₅₀ of 0.487 µM and a maximum inhibition rate of 100% (Table 5). Under the same conditions, Siponimod had an IC₅₀ greater than 10 µM and a maximum inhibition rate of 7.71% (Table 5).

In summary, compound I can effectively inhibit cytokines (CCL5) and reactive nitrogen species (NO) induced by LPS/IFNy, showing a dose-response relationship: for cytokines (CCL5), the maximum inhibition rate was 80.29% with an IC₅₀ of 0.863 µM; and for reactive nitrogen species (NO), the maximum inhibition rate was 100.00% with an IC₅₀ of 0.487 µM. The IC₅₀ of Siponimod in this experiment was consistently greater than 10 µM.

Compound I can significantly inhibit the release of pro-inflammatory factors caused by the excessive activation of microglia (BV-2), which suggests it is one of the important mechanisms for the treatment of neuroinflammatory diseases such as multiple sclerosis.

**Example 2:** Study on *in vivo* pharmacodynamics of compound I on MOG35-55-induced experimental autoimmune encephalomyelitis (EAE) mouse model

The experimental autoimmune encephalomyelitis (EAE) mouse model is an ideal animal model for researching multiple sclerosis.

Experimental objective: To study the efficacy of compound I in the MOG35-55-induced experimental autoimmune encephalomyelitis mouse model.

### Experimental design:

(1) Animals: C57BL/6 mice, female, 6 to 8 weeks old, specific pathogen-free (SPF), provided by Shanghai SLAC Laboratory Animal Co., Ltd.
(2) Test compounds and reagents:
   1) MOG35-55 was purchased from GL Biochem, Catalog No.: 51716; 2) IFA was purchased from Sigma-Aldrich, Catalog No.: F5506; 3) Mycobacterium tuberculosis H37Ra was purchased from Difco, Catalog No.: 231141; 4) PTX (Pertussis toxin) was purchased from List Biological Laboratories, Catalog No.: 180235AIA; 5) Dexamethasone acetate tablets were provided by Cisen Pharmaceutical, National Medicine Permission No.: H37021898; 6) Preparation method for dexamethasone acetate tablets: Dexamethasone acetate tablets were dissolved in saline; 7) Compound I was provided by Guangzhou JOYO Pharmatech Co., Ltd.

### Preparation method of compound I:

An appropriate amount of compound I was weighed and directly dissolved in a vehicle containing 0.5% (w/v) hydroxypropyl methylcellulose (HPMC) E5, 0.5% (w/v) polyvinylpyrrolidone (PVP), and 0.2% (w/v) sodium dodecyl sulfate (SDS) aqueous solution. The mixture was subjected to ultrasonication and stirring for approximately 1 hour at room temperature to obtain a uniform, opaque suspension of fine particles. The preparation was done every three days and stored at 4°C.

### (3) EAE modeling process:

1) After the adaptation period, ear tags were applied to mark the mice, and the fur on the back of the mice was shaved. Three sterile glass vials and dispersion blades for a homogenizer were prepared 1 to 2 days in advance.
2) Immunogenic emulsion (containing 1 mg/mL MOG35-55 and 2 mg/mL Mycobacterium tuberculosis) was prepared. PTX solution was also prepared (each vial contained 50 µg of PTX powder, which was diluted in 1 mL of PBS to make a 50 µg/mL stock solution, and was further diluted to 1 µg/mL using PBS right before use).
3) Animal immunization
   A total of 85 mice were used. 5 mice were randomly selected as the normal control group. The remaining 80 mice were anesthetized with isoflurane and subcutaneously injected with 100 µL of immunogenic emulsion (containing 1 mg/mL MOG35-55 and 2 mg/mL Mycobacterium tuberculosis in IF A). The injections were administered at three different sites, including the neck and both sides of the groin, with approximately 33 µL of emulsion at each site. The day the animals were injected with the emulsion was defined as day 0. 0 hours (on day 0) and 48 hours (on day 2) after the emulsion injection, each mouse was intraperitoneally injected with 200 ng (i.e., 200 µL) of PTX solution (1 µg/mL).

### (4) Grouping and administration

**Table 6: Grouping and dose design**

| **Group** | **N ^{c}** | **Test substance** | **Dose (mg/kg)** | **Administration volume (mL/kg)** | **Concentration (mg/mL)** | **Route of administration** | **Administration timing ^{d,e}** |
|---|---|---|---|---|---|---|---|
| G1: Normal control group (Normal) | 5 | Vehicle ^{a} | NA | *10* | *NA* | *Oral* | Twice a day, 14 days |
| G2: Vehicle control group (Veh) | 10 | Vehicle ^{a} | NA | *10* | *NA* | *Oral* | Twice a day, 14 days |
| G3: Dexamethasone group (DEX) | 10 | Dexamethas one ^{b} | 1 | *10* | *0*.*1* | *Oral* | Once a day, 14 days Vehicle control group, 14 days |
| G4: Fingolimod group | 10 | Fingolimod ^{b} | 0.5 | *10* | *0.05* | *Oral* | Once a day, 14 days Vehicle control group, 14 days |
| G5: Compound I 3 mg/kg BID group | 10 | Compound I | 3 | *10* | *0.3* | *Oral* | Twice a day, 14 days |
| G6: Compound I 10 mg/kg BID group | 10 | Compound I | 10 | *10* | *1* | *Oral* | Twice a day, 14 days |
| G7: Compound I 20 mg/kg BID group | 10 | Compound I | 20 | *10* | *2* | *Oral* | Twice a day, 14 days |
| G8: Compound I 20 mg/kg QD group | 10 | Compound I | 20 | *10* | *2* | *Oral* | Once a day, 14 days Vehicle control group, 14 days |
| | a. The vehicle control was 0.5% HPMC E5/0.5% PVP K30/0.2% SDS aqueous solution. | | | | | | |
| | b. The vehicle was saline. | | | | | | |
| | c. 70 out of 80 model mice were selected for the efficacy trial. | | | | | | |
| | d. To ensure consistency in each group's operations, an additional vehicle was administered once a day for all once daily (QD) groups. | | | | | | |
| | e. The interval for twice daily (BID) dosing was 8 hours. | | | | | | |

### (5) Clinical assessment

The day of animal immunization was designated as day 0. Starting from day 2 to day 10, the animals' body weights were recorded three times a week. Beginning on day 11, both body weight and clinical score were recorded on a daily basis under close observation. The scoring criteria are shown in Table 7 below:

**Table 7: EAE clinical scoring criteria**

| Score | Clinical Symptoms |
|---|---|
| 0 | Normal behavior, no obvious signs of illness |
| 1 | Limp tail, unilateral hind limb weakness |
| 2 | Limp tail, bilateral hind limb weakness with unsteady gait |
| 3 | Unilateral hind limb weakness and paralysis |
| 4 | Bilateral hind limb weakness and paralysis |
| 5 | Death |

### (6) Sample collection

The endpoint sampling of the 75 animals used for efficacy evaluation was carried out on day 28, with the sampling time point being 0.25 hours after morning administration.

For each group consisting of 5 mice, euthanasia was performed followed by cardiac perfusion with saline. Subsequently, an equal volume of 10% formalin was perfused. Spinal cord tissues from the mice were collected, soaked in 10% formalin solution, and then sectioned. Hematoxylin & Eosin (H&E) staining and Luxol Fast Blue (LFB) staining were performed, along with pathological scoring. The pathological scoring criteria are shown in Table 8.

**Table 8: Spinal cord H&E and LFB pathological scoring criteria**

| **Inflammation** | |
|---|---|
| 0 | Normal |
| 1 | Inflammation of the pia mater, <5 foci/slice |
| 2 | Inflammation of the pia mater, >5 foci/slice |
| 3 | Onset of inflammation of the cerebrospinal cord parenchyma, <5 foci/slice (involvement from the pia mater to the parenchyma) |
| 4 | Inflammation of the parenchyma, <5 foci/slice |
| 5 | Inflammation of the parenchyma, >5 foci/slice (small parenchymal lesions) |
| 6 | Inflammation of the parenchyma, >5 foci/slice (large parenchymal lesions) |
| 7 | Inflammation of the parenchyma with large lesions and tissue cavitation |

| **Lesion Area** | |
|---|---|
| | Leision Area %, area of lesion involvement as a percentage of cross-section |

| **Demyelination** | |
|---|---|
| 0 | No demyelination |
| 1 | Few scattered exposed axons |
| 2 | Focal exposed axons |
| 3 | Multifocal exposed axons |
| 4 | Confluent demyelinating lesions |
| 5 | Extensive demyelination |

### (7) Statistical analysis

Experimental data were plotted using Graph Pad Prism 7 and presented as mean ± SEM (standard error of the mean). The area under the curve for the clinical scoring was analyzed using one-way analysis of variance (One-way ANOVA). A p-value of less than 0.05 was considered to be significantly different when compared with the model group.

### (8) Experimental results

### 1) Clinical scoring and incidence

**Table 9: Effect of repeated administration of compound I on EAE clinical scoring in mice (14 days of administration, from day 13 to day 27)**

| Days | G1 Normal Group | | G2 Model Group (Bid*14) | | G3 Dexamethasone (Qd+V*14) | | G4 Fingolimod 0.5 mg/kg (Qd+V*14) | | G5 Compound 13 mg/kg (Bid*14) | | G6 Compound I 10 mg/kg (Bid*14) | | G7 Compound I 20 mg/kg (Bid*14) | | G8 Compound I 20 mg/kg (Qd+V*14) | |
|---|---|---|---|---|---|---|---|---|---|---|---|---|---|---|---|---|
| | Mean | Standard error | Mean | Standard error | Mean | Standard error | Mean | Standard error | Mean | Standard error | Mean | Standard error | Mean | Standard error | Mean | Standard error |
| 0 | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 |
| 2 | 000 | 000 | 0.00 | 0.00 | 0.00 | 0.00 | 0.00 | 0.00 | 0.00 | 0.00 | 0.00 | 0.00 | 0.00 | 0.00 | 0.00 | 000 |
| 4 | 0.00 | 000 | 0.00 | 0.00 | 0.00 | 0.00 | 0.00 | 0.00 | 0.00 | 0.00 | 0.00 | 0.00 | 0.00 | 0.00 | 0.00 | 0.00 |
| 7 | 0.00 | 000 | 0.00 | 0.00 | 0.00 | 0.00 | 0.00 | 0.00 | 0.00 | 0.00 | 0.00 | 0.00 | 0.00 | 0.00 | 0.00 | 0.00 |
| 9 | 0.00 | 000 | 0.00 | 0.00 | 0.00 | 0.00 | 0.00 | 0.00 | 0.00 | 0.00 | 0.00 | 0.00 | 0.00 | 0.00 | 0.00 | 000 |
| 11 | 000 | 000 | 0.00 | 0.00 | 0.00 | 0.00 | 0.00 | 0.00 | 0.00 | 0.00 | 0.00 | 0.00 | 0.00 | 0.00 | 0.00 | 000 |
| 12 | 0.00 | 000 | 0.00 | 0.00 | 0.10 | 0.10 | 0.00 | 0.00 | 0.00 | 0.00 | 0.00 | 0.00 | 0.00 | 0.00 | 0.00 | 000 |
| 13 | 0.00 | 000 | 0.30 | 0.15 | 0.20 | 0.13 | 0.20 | 0.13 | 0.20 | 0.13 | 0.20 | 0.13 | 0.30 | 0.15 | 0.20 | 0.13 |
| 14 | 0.00 | 000 | 1.00 | 0.26 | 0.70 | 0.21 | 0.70 | 0.21 | 0.40 | 0.16 | 0.40 | 0.16 | 0.60 | 0.22 | 0.60 | 0.22 |
| 15 | 0.00 | 000 | 1.60 | 0.40 | 1.30 | 0.33 | 1.30 | 0.33 | 0.60 | 0.16* | 0.50 | 0.17** | 0.80 | 0.25 | 1.10 | 0.31 |
| 16 | 0.00 | 000 | 2.10 | 0.41 | 1.70 | 0.47* | 1.70 | 0.47 | 0.90 | 0.23** | 0.60 | 0.22**** | 0.90 | 0.28** | 1.30 | 0.40 |
| 17 | 0.00 | 000 | 2.50 | 0.34 | 1.70 | 0.47*** | 1.70 | 0.47 | 1.50 | 0.34* | 0.50 | 0.22**** | 1.00 | 0.30**** | 1.40 | 0.34** |
| 18 | 000 | 000 | 2.70 | 0.37 | 1.40 | 0.43**** | 1.40 | 0.43*** | 1.90 | 0.38 | 0.60 | 0.31**** | 1.10 | 0.23**** | 1.30 | 0.37*** |
| 19 | 000 | 000 | 300 | 0.33 | 1.30 | 0.40**** | 1.30 | 0.40**** | 1.90 | 0.41** | 0.40 | 0.31**** | 1.10 | 0.23**** | 1.10 | 0.31**** |
| 20 | 000 | 000 | 2.70 | 0.30 | 1.00 | 0.39**** | 1.00 | 0.39**** | 1.30 | 0.40*** | 0.30 | 0.3**** | 0.90 | 0.28**** | 0.80 | 0.29**** |
| 21 | 0.00 | 000 | 2.20 | 0.29 | 0.90 | 0.41**** | 0.90 | 0.41*** | 1.10 | 0.38** | 0.30 | 0.21**** | 0.80 | 0.29*** | 0.70 | 0.26**** |
| 22 | 0.00 | 000 | 1.80 | 0.20 | 0.80 | 0.39*** | 0.80 | 0.39* | 1.10 | 0.38 | 0.30 | 0.21**** | 0.50 | 0.31*** | 0.40 | 0.16*** |
| 23 | 0.00 | 000 | 1.70 | 0.21 | 0.70 | 0.40*** | 0.70 | 0.40* | 1.00 | 0.33 | 0.40 | 0.22*** | 0.50 | 0.31** | 0.40 | 0.16*** |
| 24 | 0.00 | 000 | 1.30 | 0.21 | 0.30 | 0.15* | 0.50 | 0.40 | 0.80 | 0.33 | 0.40 | 0.22* | 0.30 | 0.3* | 0.30 | 0.15* |
| 25 | 0.00 | 000 | 1.30 | 0.21 | 0.30 | 0.15* | 0.50 | 0.40 | 0.80 | 0.33 | 0.40 | 0.22* | 0.30 | 0.3* | 0.30 | 0.15* |
| 26 | 0.00 | 0.00 | 1.30 | 0.26 | 0.30 | 0.15* | 0.50 | 0.40 | 0.80 | 0.33 | 0.40 | 0.22* | 0.30 | 0.3* | 0.30 | 0.15* |
| 27 | 0.00 | 0.00 | 1.30 | 0.26 | 0.30 | 0.15* | 0.50 | 0.40 | 0.60 | 0.34 | 0.30 | 0.15* | 0.30 | 0.3* | 0.30 | 0.15* |

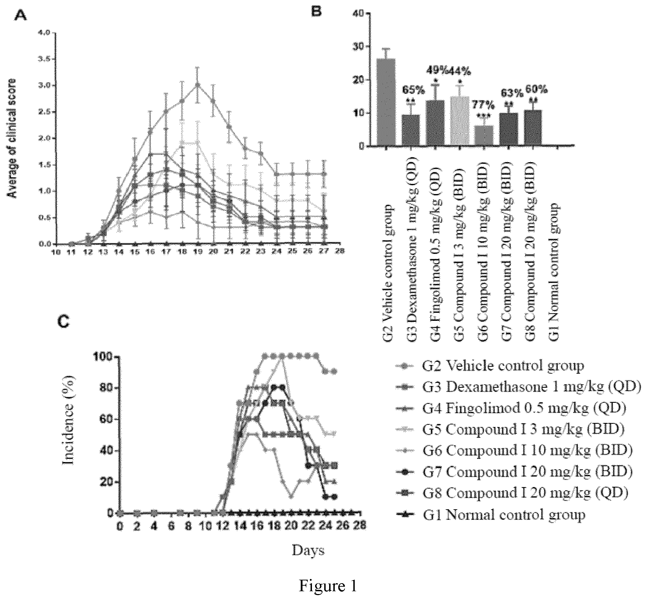

On day 12 after immunization, the mice began to show clinical symptoms. Administration started after grouping on day 13. As shown in Figure 1-A and Table 9, the average clinical score of the vehicle control group gradually increased and reached a peak on day 19 with an average score of approximately 3.0, confirming the successful establishment of the EAE model in this experiment (Figure 1-A and Table 9). Compared with the vehicle control (Veh control) group, the positive control drug Dexamethasone (DEX) had a significant inhibitory effect on disease progression. The clinical scores were significantly different from those of the vehicle control group from day 15 to day 27, with a 65% reduction rate in the area under the curve (AUC) for clinical scoring (Figure 1-B). The clinical scores for the positive control group Fingolimod were significantly different from those of the vehicle control group from day 17 to day 23, with a 49% reduction rate in the area under the curve (AUC) (Figure 1-B).

All dose groups of test compound I significantly reduced the clinical scores of EAE mice. Specifically, the 3 mg/kg twice daily (BID) group from day 15 to day 21, the 10 mg/kg BID group from day 15 to day 27, the 20 mg/kg BID group from day 16 to day 27, and the 20 mg/kg once daily (QD) group from day 17 to day 27 showed significant difference from the vehicle control group. The inhibition rates for the compound I administration groups, relative to the AUC for clinical scoring in the vehicle control group, were 44% for the 3 mg/kg twice daily (BID) group, 77% for the 10 mg/kg BID group, 63% for the 20 mg/kg BID group, and 60% for the 20 mg/kg once daily (QD) group (Figure 1-B). Therefore, compound I significantly improved the clinical symptoms in the EAE model, with an effective dose starting at 3 mg/kg twice daily (BID), and demonstrated a certain dose-response relationship. The therapeutic effect of the 10 mg/kg twice daily (BID) group was superior to that of Dexamethasone and Fingolimod, whose inhibition rates for the AUC for clinical scoring were 65% and 49%, respectively.

**Table 10: Effect of repeated administration on incidence of EAE in mice (%) (14 days of administration, from day 13 to day 27)**

| Days | G2 Vehicle control group | G3 Dexamethasone 1 mg/kg PO | G4 Fingolimod 0.5 mg/kg | G5 Compound I 3 mg/kg | G6 Compound 110 mg/kg | G7 Compound I 20 mg/kg | G8 Compound I 20 mg/kg | G1 Normal control group |
|---|---|---|---|---|---|---|---|---|
| 0 | 0 | 0 | 0 | 0 | 0 | 0 | 0 | 0 |
| 2 | 0 | 0 | 0 | 0 | 0 | 0 | 0 | 0 |
| 4 | 0 | 0 | 0 | 0 | 0 | 0 | 0 | 0 |
| 7 | 0 | 0 | 0 | 0 | 0 | 0 | 0 | 0 |
| 9 | 0 | 0 | 0 | 0 | 0 | 0 | 0 | 0 |
| 11 | 0 | 0 | 0 | 0 | 0 | 0 | 0 | 0 |
| 12 | 0 | 10 | 0 | 0 | 0 | 0 | 0 | 0 |
| 13 | 30 | 20 | 20 | 20 | 20 | 30 | 20 | 0 |
| 14 | 70 | 60 | 60 | 40 | 40 | 50 | 50 | 0 |
| 15 | 70 | 60 | 80 | 60 | 50 | 60 | 70 | 0 |
| 16 | 90 | 60 | 80 | 70 | 50 | 60 | 70 | 0 |
| 17 | 100 | 50 | 80 | 80 | 40 | 70 | 80 | 0 |
| 18 | 100 | 50 | 70 | 90 | 40 | 80 | 70 | 0 |
| 19 | 100 | 50 | 70 | 100 | 20 | 80 | 70 | 0 |
| 20 | 100 | 50 | 60 | 70 | 10 | 70 | 50 | 0 |
| 21 | 100 | 50 | 50 | 60 | 20 | 60 | 50 | 0 |
| 22 | 100 | 40 | 50 | 60 | 20 | 30 | 40 | 0 |
| 23 | 100 | 30 | 40 | 60 | 30 | 30 | 40 | 0 |
| 24 | 90 | 30 | 20 | 50 | 30 | 10 | 30 | 0 |
| 25 | 90 | 30 | 20 | 50 | 30 | 10 | 30 | 0 |
| 26 | 90 | 30 | 20 | 50 | 30 | 10 | 30 | 0 |
| 27 | 90 | 30 | 20 | 30 | 30 | 10 | 30 | 0 |

Moreover, the incidence results showed (Table 10) that the vehicle control group had a 100% incidence on day 17, while the positive control groups with Dexamethasone (DEX) and Fingolimod had reduced incidence (Figure 1-C). The compound I administration groups all showed delayed onset and reduced incidence. Among them, the 10 mg/kg twice daily (BID) group had the lowest incidence; the 20 mg/kg twice daily (BID) group showed a sustained advantage in inhibiting the incidence in the later stages of the disease (Figure 1-C).

### 2) Spinal cord pathological analysis

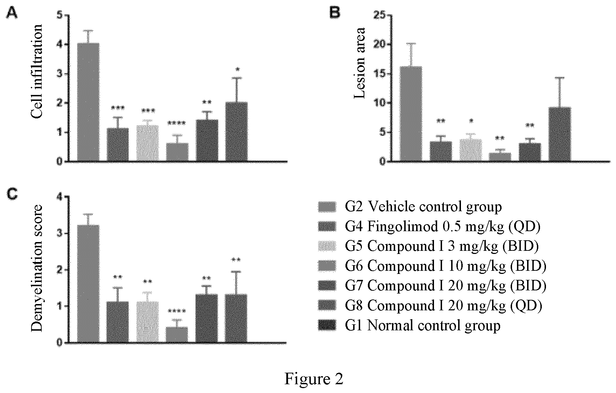

At the end of the experiment, the spinal cords of mice were collected, with 2 in the normal control group and 5 in each of the remaining groups. The spinal cords were fixed in formalin, followed by paraffin embedding and sectioning. Two sections were taken from each mouse and subjected to H&E and FLB staining for pathological observation. Scoring was done for cell infiltration, lesion areas, and demyelination. The experiment showed that the model vehicle control (Veh) group exhibited significant pathological damage, with inflammatory cell infiltration and multifocal exposed axons in the spinal cord white matter. Specifically, as shown in Figure 2, the model group had a cell infiltration score of 4, a lesion area of 15%, and a demyelination score of 3.2. Consistent with the EAE clinical scoring results, test compound I at different doses all significantly inhibited the damage to the spinal cord caused by EAE modeling. Among them, the 10 mg/kg twice-daily (BID) group showed a significantly better therapeutic effect than the positive drug Fingolimod group (Figure 2) in terms of cell infiltration (Figure 2-A), lesion area (Figure 2-B), and demyelination (Figure 2-C). Specifically, after H&E staining of the mouse spinal cord, cell infiltration (A) and lesion area (B) in the spinal cord sections were counted. After LFB staining, the degree of demyelination (C) in the spinal cord sections was scored. Data were presented as mean ± standard error of the mean (mean ± SEM). *p<0.05, **p<0.01, ***p<0.001, ****p<0.0001, vs. vehicle control group (Vehicle), one-way ANOVA.

The pathological analysis results showed that all dose groups of compound I improved the pathological changes caused by the EAE model. Specifically, the group treated with 10 mg/kg of compound I twice a day (BID) showed better improvement in terms of inflammatory cell infiltration, lesion area, and demyelination compared to the positive control group treated with Fingolimod.

In the present disclosure, the results demonstrate a good therapeutic effect on the experimental autoimmune encephalomyelitis (EAE) mouse model. The present disclosure provides a new medicament for treating multiple sclerosis and encephalomyelitis and holds great clinical application value.

**Example 3:** All dose groups of compound I significantly reduced the level of STATS phosphorylation in spinal cord
(1) Animals: C57BL/6 mice, female, 6 to 8 weeks old, specific pathogen-free (SPF), provided by Shanghai SLAC Laboratory Animal Co., Ltd.
(2) Test compounds and reagents:
   MOG35-55 was purchased from GL Biochem, Catalog No.: 51716;
   IFA was purchased from Sigma-Aldrich, Catalog No.: F5506;
   Mycobacterium tuberculosis H37Ra was purchased from Difco, Catalog No.: 231141;
   PTX (Pertussis toxin) was purchased from List Biological Laboratories, Catalog No.: 180235AIA;
   Dexamethasone acetate tablets were provided by Cisen Pharmaceutical, National Medicine Permission No.: H37021898;
   Preparation method for Dexamethasone acetate tablets: Dexamethasone acetate tablets were dissolved in saline.

Compound I was provided by Guangzhou JOYO Pharmatech Co., Ltd.

### Preparation method of compound I:

An appropriate amount of compound I was weighed and directly dissolved in a vehicle containing 0.5% (w/v) hydroxypropyl methylcellulose (HPMC) E5, 0.5% (w/v) polyvinylpyrrolidone (PVP), and 0.2% (w/v) sodium dodecyl sulfate (SDS) aqueous solution. The mixture was subjected to ultrasonication and stirring for approximately 1 hour at room temperature to obtain a uniform, opaque suspension of fine particles. The preparation was done every three days and stored at 4°C.

### (3) EAE modeling process:

1) After the adaptation period, ear tags were applied to mark the mice, and the fur on the back of the mice was shaved. Three sterile glass vials and dispersion blades for a homogenizer were prepared 1 to 2 days in advance.
2) Immunogenic emulsion (containing 1 mg/mL MOG35-55 and 2 mg/mL Mycobacterium tuberculosis) was prepared. PTX solution was also prepared (each vial contained 50 µg of PTX powder, which was diluted in 1 mL of PBS to make a 50 µg/mL stock solution, and was further diluted to 1 µg/mL using PBS right before use).
3) Animal immunization
   A total of 85 mice were used. 5 mice were randomly selected as the normal control group. The remaining 80 mice were anesthetized with isoflurane and subcutaneously injected with 100 µL of immunogenic emulsion (containing 1 mg/mL MOG35-55 and 2 mg/mL Mycobacterium tuberculosis in IF A). The injections were administered at three different sites, including the neck and both sides of the groin, with approximately 33 µL of emulsion at each site. The day the animals were injected with the emulsion was defined as day 0. 0 hours (on day 0) and 48 hours (on day 2) after the emulsion injection, each mouse was intraperitoneally injected with 200 ng (i.e., 200 µL) of PTX solution (1 µg/mL).

### (4) Grouping and administration

**Table 11: Grouping and dose design**

| Group | N ^{c} | Test substance | Dose (mg/kg) | Administration volume (mL/kg) | Concentration (mg/mL) | Route of administration | Administration timing ^{d,e} |
|---|---|---|---|---|---|---|---|
| G1: Normal control group (Normal) | 5 | Vehicle ^{a} | NA | 10 | NA | Oral | Twice a day, 14 days |
| G2: Vehicle control group (Veh) | 10 | Vehicle ^{a} | NA | 10 | NA | Oral | Twice a day, 14 days |
| G3: Dexamethasone group (DEX) | 10 | Dexamethasone ^{b} | 1 | 10 | 0.1 | Oral | Once a day, 14 days |
| | | | | | | | Vehicle control group, 14 days |
| G4: Fingolimod group | 10 | Fingolimod ^{b} | 0.5 | 10 | 0.05 | Oral | Once a day, 14 days |
| | | | | | | | Vehicle control group, 14 days |
| G5: Compound I 3 mg/kg BID group | 10 | Compound I | 3 | 10 | 0.3 | Oral | Twice a day, 14 days |
| G6: Compound I 10 mg/kg BID group | 10 | Compound I | 10 | 10 | 1 | Oral | Twice a day, 14 days |
| G7: Compound I 20 mg/kg BID group | 10 | Compound I | 20 | 10 | 2 | Oral | Twice a day, 14 days |
| G8: Compound I 20 mg/kg QD group | 10 | Compound I | 20 | 10 | 2 | Oral | Once a day, 14 days |
| | | | | | | | Vehicle control |
| | | | | | | | group, 14 days |
| | a. The vehicle control was 0.5% HPMC E5/0.5% PVP K30/0.2% SDS aqueous solution. | | | | | | |
| | b. The vehicle was saline. | | | | | | |
| | c. 70 out of 80 model mice were selected for the efficacy trial. | | | | | | |
| | d. To ensure consistency in each group's operations, an additional vehicle was administered once a day for all once daily (QD) groups. | | | | | | |
| | e. The interval for twice daily (BID) dosing was 8 hours. | | | | | | |

### (5) Sample collection and testing

For efficacy evaluation, samples from 75 animals (of which, 5 served as a normal control group, and 70 mice were selected from 80 model mice for the efficacy trial) were collected at the endpoint on day 28, with the sampling time point set at 0.25 hours after morning administration. The following samples were collected:

From each group of 5 animals, blood was drawn from the heart after anesthesia with isoflurane, without performing perfusion, and then the animals were euthanized for sampling.

The spinal cord was collected and stored directly at -80°C for Western blot experiments to detect the protein expression levels of STATS, pSTAT3, and GAPDH.

### (6) Experimental results

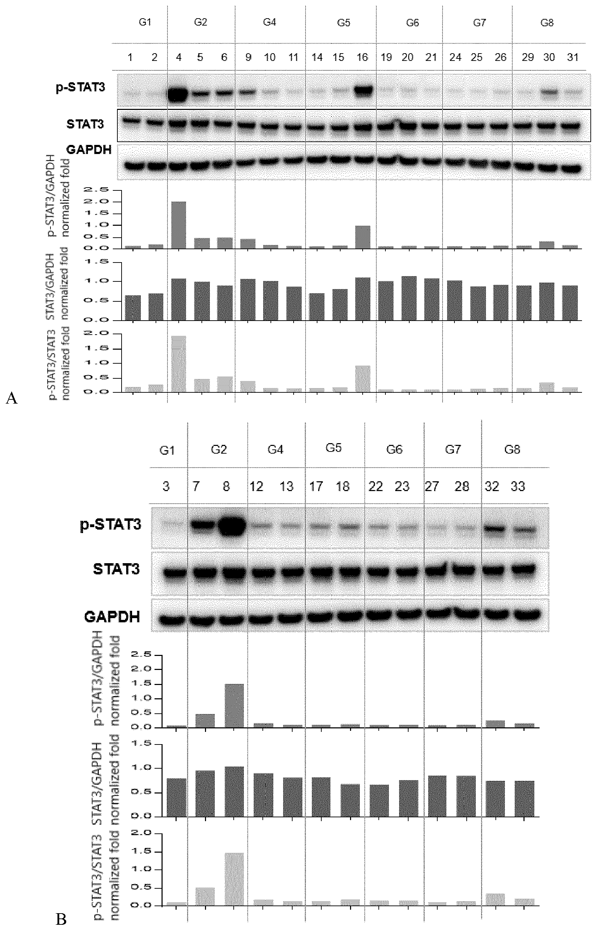

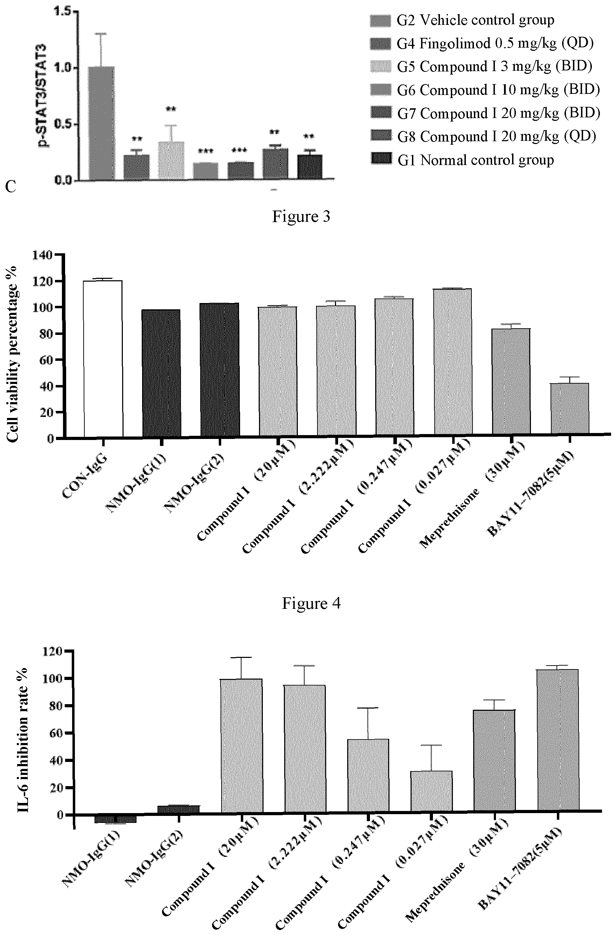

Samples were collected at the endpoint 0.25 hours after morning administration on day 28 of the experiment. Mouse spinal cord samples were collected from the normal control group (n = 3), the vehicle control group (n = 5), the positive control Fingolimod group (n = 5), and all compound I administration groups (n = 5 for each group). The protein levels of STATS, pSTAT3, and GAPDH were detected by Western blot, and band quantification was performed using GE Healthcare 1D Quant Densitometry software. The level of STATS phosphorylation (p-STAT3/STAT3) reflected the level of inflammatory cell infiltration and inflammation response in the spinal cord. Compared to the normal group, there was a significant elevation in STAT3 phosphorylation in the spinal cord samples of the EAE model vehicle control group (Figures 3-A and 3-B). As shown in Figures 3-A and 3-B, the positive control drug, Fingolimod, significantly inhibited STAT3 phosphorylation to normal animal levels. Consistent with the clinical scores, all dose groups of test compound I significantly inhibited the level of STAT3 phosphorylation in spinal cord. Among them, the 10 mg/kg BID and 20 mg/kg BID groups had the best inhibitory effect, with an inhibition rate reaching 86% (Figure 3-C). Data are presented as mean ± standard error of the mean (mean ± SEM). **p<0.01, ***p<0.001, vs. vehicle control group (Vehicle), one-way ANOVA.

Conclusion: All dose groups of compound I significantly reduced the level of STATS phosphorylation in spinal cord. Among them, the 10 mg/kg BID and 20 mg/kg BID groups had the best inhibitory effect, with an inhibition rate of approximately 86%.

**Example 4:** IC₅₀ values (nM) of compound I for inhibiting pSTAT1 and pSTAT5 in hPBMC

### 1. Cells

**Table 12: Cells**

| Name | Supplier | Catalog or item number | Batch number |
|---|---|---|---|
| hPBMC | stemcell | 70025.3 | 2009428002 |

### 2. Main reagents

**Table 13: Main reagents**

| **Name** | **Supplier** | **Catalog or item number** | **Storage conditions** |
|---|---|---|---|
| Human IL-6 | absin | abs00808 | -807 |
| Human IL-2 | absin | abs00804 | -807 |
| Human pSTAT1 antibody | BD | 612564 | 41 |
| Human pSTAT5 antibody | BD | 562077 | 42 |
| Fixation solution | BD | 558049 | 43 |
| Permeabilization solution | BD | 558050 | -202 |

Specifically, pSTAT1 refers to phosphorylated STAT1, and pSTAT5 refers to phosphorylated STATS.

### 3. Experimental methods

### 1. Drug incubation and cytokine stimulation

1) Preparation of culture medium: 1640 medium + 10% fetal bovine serum + 1% penicillin/streptomycin + 1% non-essential amino acids (all percentages were v/v)
2) Cells were thawed in a 37°C water bath and then centrifuged. The cell concentration was adjusted to 5×10⁵ cells/mL using the prepared culture medium.
3) Cell suspension was seeded into a 96-well round-bottom plate at 200 µL/well and incubated at 37°C for 90 minutes.
4) Drugs were added, followed by a 37°C incubation for 30 minutes.
5) Cells were then stimulated by adding 10 ng/mL of IL-6 or 4 ng/mL of IL-2, followed by a 37°C incubation for 15 minutes.

### 4. Cell staining and testing

1) After completing the stimulation, cells were centrifuged and washed once with staining buffer (DPBS + 2% bovine serum albumin). Antibodies were then added for a 30-minute staining process at 4°C (CD4 antibody was added for cells stimulated with IL-6 and IL-2).
2) Cells were washed twice with staining buffer and then fixed with fixation solution for a 20-minute fixing process at 4°C.
3) Cells were washed once with staining buffer before adding -20°C pre-chilled permeabilization solution for a 30-minute permeabilization process at 4°C.
4) After washing the cells twice with staining buffer, antibodies were added for another 30-minute staining process at 4°C (IL-6 and IL-2).
5) Cells were washed twice with staining buffer and resuspended. Flow cytometry was then used to measure the MFI values of pSTAT1 or pSTAT5 in CD4 or CD33 positive cells.

### 5. Data analysis

1) Response% = [(MFI value of drug-treated well - mean MFI value of control well)/(mean MFI value of DMSO well - mean MFI value of control well)] * 100% was calculated.
2) The calculated Response% values were input into GraphPad Prism 8 software. Inhibition curves were plotted using the "log(inhibitor) vs. response -- Variable slope (four parameters)" method, and the corresponding IC₅₀ values for the samples were obtained.

### 5. Experimental results

Compound I exhibited inhibitory activity on the levels of IL-6-induced pSTAT1 and IL-2-induced pSTAT5 in hPBMCs. The IC₅₀ values for the inhibition were 73.1 nM and 36.3 nM, respectively (Table 14).

**Table 14: IC₅₀ values (nM) of drugs for inhibition of pSTAT1 and pSTAT5**

| Group | | IC₅₀ value (nM) |
|---|---|---|
| 01 | IL-6-induced pSTAT1 | 73.1 |
| 02 | IL-2-induced pSTAT5 | 36.3 |

Specifically, IL-2 refers to interleukin-2; IL-6 refers to interleukin-6.

Conclusion: Compound I exhibited inhibitory activity on the levels of pSTAT1 and pSTAT5 in hPBMCs.

### Example 5

Experimental objective: To study the inhibitory effect of compound I on IL-6 secretion in NMO-IgG induced astrocyte injury model

### Experimental design

1. Animals: Pregnant Wistar rats were purchased from Beijing Vital River Laboratory Animal Technology Co., Ltd.
2. Information on test sample and control

**Table 15: Information on test sample and positive control**

| Compound | Catalog number | Batch number | Purity (%) | Molecular weight | Storage conditions | Supplier |
|---|---|---|---|---|---|---|
| Compound I | NA | CPo126337-01-04P-01-68-01 | 99.7 | 293 | Room temperature, sealed | Provided by Guangzhou JOYO Pharmatech Co., Ltd. |
| Meprednisone | T6580 | 117788 | 98.07 | 372.46 | -20°C | Topscience Co., Ltd. |
| BAY11-7082 | T1902 | 147885 | 97.91 | 207.25 | -20°C | Topscience Co., Ltd. |

The compound was dissolved in DMSO to prepare a compound stock solution. The compound stock solution was then serially diluted in a gradient using DMSO to prepare a series of 1000X test compound solutions. These 1000X test compound solutions were further diluted at a ratio of 1:100 with cell culture medium to prepare a series of 10X test compound solutions. Finally, according to the experimental well plate loading design, a corresponding volume of 10X solution was added to each well, with two duplicate wells for each drug concentration. In the experiment, the test solution (i.e., 1X solution) had varying final concentrations of compound I, specifically 20, 2.222, 0.247, and 0.027 µM; the concentration of Meprednisone compound (i.e., 1X solution) was 30 µM; the concentration of BAY 11-7082 (i.e., 1X solution) was 5 µM; the final DMSO concentration in each well was 0.1%. DMSO without compound was added to blank control wells, with a final concentration of 0.1%.

### 3. Experimental steps:

### (1) Extraction, separation, and culture of rat primary astrocytes

1) Extraction and separation of primary astrocytes: A mixed glial cell culture medium was prepared from the cerebral cortex of 1-day-old newborn rats. After euthanizing the newborn rats, they were immersed in alcohol. Their skin was cut, the brains were removed, and the cerebral cortex was isolated. The tissues were then ground to remove the connective tissue, and the mixed glial cells were sieved through a 70 µm cell strainer. These cells were then seeded into a cell culture plate coated with poly-L-lysine. The culture medium was DMEM, containing 10% heat-inactivated fetal bovine serum, 100 U/mL penicillin, and 100 mg/mL streptomycin. After about 12 days of *in vitro* culture, the cells reached confluence. Microglia, endothelial cells, and oligodendrocytes were removed from the mixed glial cells by vibratory shaking. The purity of the astrocytes was subsequently improved through two rounds of trypsin digestion and seeding, achieving a purity of >95%.
2) Culture of astrocytes: The isolated astrocytes were cultured in a 37°C, 5% CO₂ incubator. The culture medium was changed every 3 to 4 days. The astrocytes could also be digested using trypsin (0.25%) and then frozen in liquid nitrogen for future use.

### (2) Acquisition, isolation, and purification of NMO-IgG

1) Twelve serum samples from NMOSD patients who tested positive for AQP4-IgG and met the latest international diagnostic criteria for NMOSD revised in 2015 were collected (Ethics approval number: KY 2021-069-01). All patients meeting the diagnostic criteria for NMOSD were determined negative and positive by the currently internationally recognized indirect immunofluorescence staining method (i.e., cell-based assay (CBA)) for the detection of serum AQP4-IgG antibodies. The serum samples were 0.22 µm sterile filtered and stored at -80°C. Before purification, they were inactivated at 56°C for 30 minutes.
2) Antibody (IgG) purification in serum

Human NMO-IgG was isolated from a sterile-filtered serum pool (AQP4-IgG-positive NMOSD) using a HiTrap Protein G-HP column (GE Healthcare). The serum samples were diluted at a ratio of 1:1 with binding buffer, and after packing the purification column with binding buffer, the serum sample was added. The column was subsequently washed with binding buffer again and the antibodies were eluted according to the instructions. Finally, the antibodies were concentrated on an Amicon Ultra-4 centrifugal filter (millipore) with a 10,000 MW cutoff. The concentrated immunoglobulin was filtered through a 0.22 µm filter. The protein concentration of the concentrate was determined using the BCA method and stored at -80°C. The total IgG extracted from the serum of NMOSD patients was NMO-IgG (containing AQP4-IgG), and the cells were stimulated at a protein concentration of 250 µg/mL during the experiment.

### 3) Control-IgG:

Control-IgG (Jackson ImmunoResearch, 009-000-002) was a commercial reagent. The stock solution had a concentration of 11.5 mg/mL, and it was used at a working concentration of 250 µg/mL during the experiment.

### (3) Drug incubation and sample testing

### 1) Drug incubation

Astrocytes were cultured or revived until day 2 and then seeded into a 24-well plate at a density of 1.5 × 10⁵ cells per well. The test compounds, including compound I, Meprednisone, and BAY11-7082, were pre-incubated with the cells for 30 minutes at the concentrations previously described. Subsequently, in each group, the cells continued to be stimulated with NMO-IgG at a final working concentration of 250 µg/mL for 24 hours to induce astrocyte damage. Normal human Control-IgG (CON-IgG) stimulation at a final working concentration of 250 µg/mL was used as a negative control.

### 2) Sample collection and testing

After 24 hours of NMO-IgG stimulation of cells, the supernatant was collected. IL-6 levels were detected using an enzyme-linked immunosorbent assay (ELISA) following the instructions of the IL-6 ELISA kit (R&D Systems, USA, #RD6000B). The absorbance (OD values) of the samples in each group was measured, and the concentration of IL-6 in each experimental group was calculated through standard curve fitting. Cell viability for each group was assessed using the CCK-8 method after the supernatant had been removed from the cell culture plate. Following the instructions of the CCK-8 kit (Dojindo Laboratories, #CK04), the absorbance (OD values) of the cells in each group was measured. The cell viability for each experimental group was then calculated upon the conclusion of the incubation.

### (5) Data analysis

1) Cell viability percentage: Through the CCK8 optical density values (OD values), the cell viability percentage (%) at various test concentrations relative to the blank control group (NMO-IgG + DMSO) was calculated. The calculation formula was as follows: cell viability percentage (%) = (OD value of test concentration well - blank plate background value)/(OD value of blank control group - blank plate background value) × 100.
2) IL-6 inhibition rate:

Firstly, standard curves were plotted based on the ELISA optical density values, and the absolute concentration of each sample was calculated.

The relative inhibition percentage of IL-6 secretion at various test concentrations relative to the blank control group (NMO-IgG + DMSO), i.e., IL-6 inhibition rate (%), was then calculated based on the absolute concentration of each sample. The calculation formula was as follows: IL-6 inhibition rate (%) = (absolute concentration of blank control group - absolute concentration of test concentration well)/(absolute concentration of blank control group - absolute concentration of negative control well) × 100.

### (6) Experimental results

The CCK8 experimental results indicated that in the NMO-IgG induced astrocyte injury model, as the test compound, compound I showed no inhibitory effect on cell viability after 24 hours of co-incubation with cells (Figure 4 and Table 16). The highest test concentration was 20 µM. Meprednisone tended to inhibit cell viability at a test concentration of 30 µM. The BAY compound significantly reduced the cell viability percentage to 39.5% at a test concentration of 5 µM.

**Table 16: Effect of compound I and control compounds on astrocyte viability**

| Cell viability percentage% | CON-IgG (negative control well) | NMO-IgG1 (blank control well) | NMO-IgG2 (blank control well) | Compound I (20 µM) | Compound I (2.222 µM) | Compound I (0.247 µM) | Compound I (0.027 µM) | Mepredni sone (30 µM) | BAY 11-7082 (5 µM) |
|---|---|---|---|---|---|---|---|---|---|
| Mean | 120.5 | 97.7 | 102.3 | 99.4 | 99.7 | 105.0 | 111.7 | 81.7 | 39.5 |
| Standard error | 1.4 | 0.1 | 0.1 | 0.8 | 3.3 | 1.1 | 1.0 | 3.0 | 4.4 |

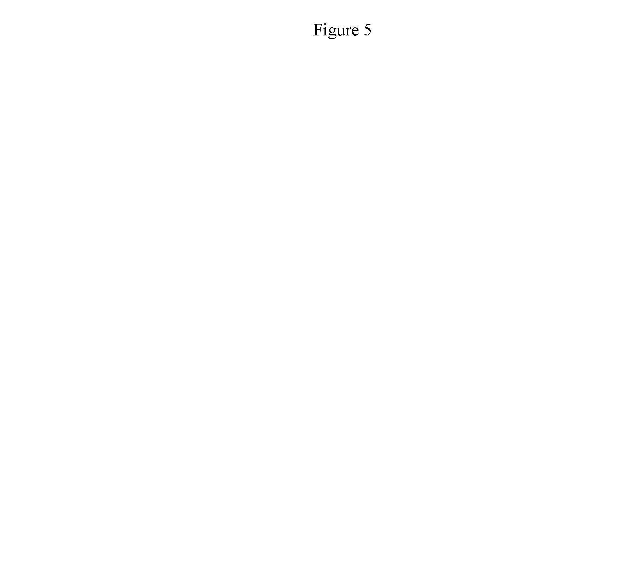

The IL-6 ELISA results indicated that compound I had a significant inhibitory effect on IL-6 release caused by NMO-IgG in astrocytes (Figure 5 and Table 17). At a test concentration of 20 µM, the inhibition rate was approximately 98.8%. Meprednisone had an inhibition rate of approximately 74.8% on IL-6 at a test concentration of 30 µM. The BAY compound had an inhibition rate of approximately 104.2% on IL-6 at a test concentration of 5 µM.

**Table 17: Effect of compound I and control compounds on IL-6 secretion by astrocytes**

| IL-6 inhibition rate% | NMO-IgG1 (blank control well) | NMO-IgG2 (blank control well) | Compound I (20 µM) | Compound I (2.222 µM) | Compound I (0.247 µM) | Compound I (0.027 µM) | Mepredni sone (30 µM) | BAY 11-7082 (5 µM) |
|---|---|---|---|---|---|---|---|---|
| Mean | -6.3 | 6.3 | 98.8 | 94.3 | 54.0 | 30.4 | 74.8 | 104.2 |
| Standard error | 0.3 | 0.3 | 15.7 | 13.9 | 23.0 | 18.7 | 7.4 | 2.6 |

Although the foregoing describes specific embodiments of the present disclosure, it should be understood by those skilled in the art that these are provided only as examples. Various changes or modifications can be made to these embodiments without departing from the principles and essence of the present disclosure. Therefore, the scope of protection for the present disclosure is defined by the appended claims.

## Claims

1. A use of a compound of formula I or a pharmaceutically acceptable salt thereof in the manufacture of a STAT phosphorylation inhibitor:

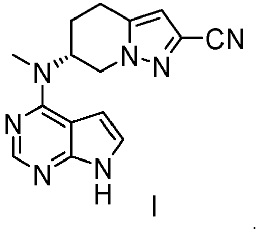

2. A use of a compound of formula I or a pharmaceutically acceptable salt thereof in the manufacture of a medicament for treating and/or preventing purulent myelitis, acute myelitis, or encephalomyelitis: 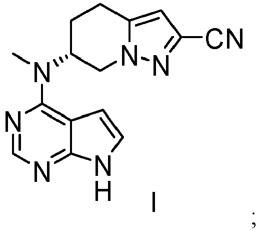 the encephalomyelitis may be acute disseminated encephalomyelitis, subacute necrotizing myelitis, acute necrotizing hemorrhagic encephalomyelitis, pediatric acute disseminated encephalomyelitis, or tuberculous myelitis.

3. A use of a compound of formula I or a pharmaceutically acceptable salt thereof in the manufacture of a medicament for treating and/or preventing an autoimmune disease: 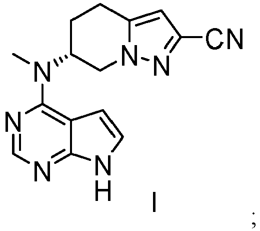 the autoimmune disease may be an autoimmune disease of the central nervous system or an autoimmune disease of the optic nervous system; the autoimmune disease of the central nervous system may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

4. A use of a compound of formula I or a pharmaceutically acceptable salt thereof in the manufacture of a medicament for treating and/or preventing purulent myelitis, acute myelitis, or encephalomyelitis **characterized by** abnormally elevated levels of STAT phosphorylation: 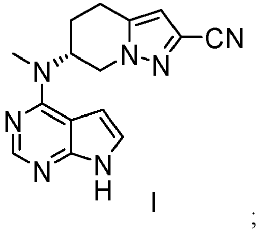 the encephalomyelitis **characterized by** abnormally elevated levels of STAT phosphorylation may be acute disseminated encephalomyelitis, subacute necrotizing myelitis, acute necrotizing hemorrhagic encephalomyelitis, pediatric acute disseminated encephalomyelitis, or tuberculous myelitis.

5. A use of a compound of formula I or a pharmaceutically acceptable salt thereof in the manufacture of a medicament for treating and/or preventing an autoimmune disease **characterized by** abnormally elevated levels of STAT phosphorylation: 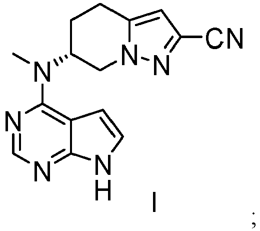 the autoimmune disease **characterized by** abnormally elevated levels of STAT phosphorylation may be an autoimmune disease of the central nervous system or an autoimmune disease of the optic nervous system; the autoimmune disease of the central nervous system **characterized by** abnormally elevated levels of STAT phosphorylation may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

6. A method for treating and/or preventing purulent myelitis, acute myelitis, or encephalomyelitis in a subject, comprising administering to the subject a therapeutically effective amount of a compound of formula I or a pharmaceutically acceptable salt thereof; 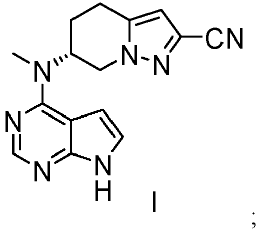 the encephalomyelitis may be acute disseminated encephalomyelitis, subacute necrotizing myelitis, acute necrotizing hemorrhagic encephalomyelitis, pediatric acute disseminated encephalomyelitis, or tuberculous myelitis.

7. A method for treating and/or preventing an autoimmune disease in a subject, comprising administering to the subject a therapeutically effective amount of a compound of formula I or a pharmaceutically acceptable salt thereof; 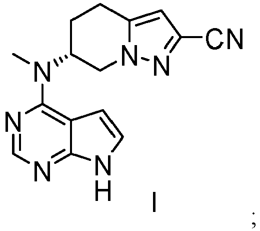 wherein the autoimmune disease may be an autoimmune disease of the central nervous system or an autoimmune disease of the optic nervous system; the autoimmune disease of the central nervous system may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

8. A method for inhibiting STAT phosphorylation in a subject, comprising administering to the subject a therapeutically effective amount of a compound of formula I or a pharmaceutically acceptable salt thereof; the STAT phosphorylation may be one or more than one of STAT1 phosphorylation, STATS phosphorylation, and STATS phosphorylation;

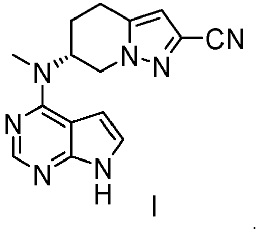

9. A method for treating and/or preventing purulent myelitis, acute myelitis, or encephalomyelitis, as well as an autoimmune disease **characterized by** abnormally elevated levels of STAT phosphorylation in a subject, comprising administering to the subject a therapeutically effective amount of a compound of formula I or a pharmaceutically acceptable salt thereof; 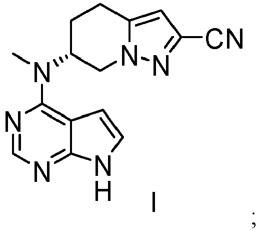 the encephalomyelitis **characterized by** abnormally elevated levels of STAT phosphorylation may be acute disseminated encephalomyelitis, subacute necrotizing myelitis, acute necrotizing hemorrhagic encephalomyelitis, pediatric acute disseminated encephalomyelitis, or tuberculous myelitis.

10. A method for treating and/or preventing an autoimmune disease **characterized by** abnormally elevated levels of STAT phosphorylation in a subject, comprising administering to the subject a therapeutically effective amount of a compound of formula I or a pharmaceutically acceptable salt thereof; 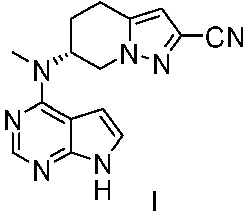 wherein the autoimmune disease **characterized by** abnormally elevated levels of STAT phosphorylation may be an autoimmune disease of the central nervous system or an autoimmune disease of the optic nervous system; the autoimmune disease of the central nervous system **characterized by** abnormally elevated levels of STAT phosphorylation may be neuromyelitis optica or multiple sclerosis, such as neuromyelitis optica, relapsing-remitting multiple sclerosis, secondary progressive multiple sclerosis, primary progressive multiple sclerosis, progressive-relapsing multiple sclerosis, clinically isolated syndrome, or radiologically isolated syndrome; the autoimmune disease of the optic nervous system may be neuromyelitis optica.

11. The use according to at least one of claims 1, 4, and 5, or the method according to at least one of claims 8, 9, and 10, wherein the STAT phosphorylation is one or more than one of STAT1 phosphorylation, STATS phosphorylation, and STATS phosphorylation.

12. The use according to at least one of claims 1 to 5, or the method according to at least one of claims 6 to 10, wherein the use or the method satisfies one or more than one of the following conditions:
(1) the compound of formula I or the pharmaceutically acceptable salt thereof is one of the active ingredients or the sole active ingredient of the inhibitor according to claim 1 or the medicament according to any one of claims 2 to 5;
(2) the compound of formula I or the pharmaceutically acceptable salt thereof is administered orally to the subject;
(3) the compound of formula I or the pharmaceutically acceptable salt thereof comprises a pharmaceutically acceptable excipient;
(4) the compound of formula I or the pharmaceutically acceptable salt thereof is administered at a dose of 1 to 120 mg/kg/d, for example, 3 to 100 mg/kg/d, for another example, 3 mg/kg/d, 6 mg/kg/d, 10 mg/kg/d, 20 mg/kg/d, 40 mg/kg/d, 60 mg/kg/d, or 90 mg/kg/d;
(5) the compound of formula I or the pharmaceutically acceptable salt thereof is administered at a single dose of 1 to 400 mg, for example, 3 3 mg, 6 mg, 9 mg, 100 mg, or 400 mg;
(6) the compound of formula I or the pharmaceutically acceptable salt thereof is administered at a frequency of once a day, twice a day, or three times a day, for example, once a day or twice a day;
(7) the compound of formula I or the pharmaceutically acceptable salt thereof is formulated into a tablet dosage form or a subcutaneous injection dosage form.

13. A pharmaceutical composition for use in treating and/or preventing purulent myelitis, acute myelitis, encephalomyelitis, or an autoimmune disease in a subject, comprising a compound of formula I or a pharmaceutically acceptable salt thereof, as well as a pharmaceutical excipient;
